# EUROPEAN PATENT APPLICATION

(11) **EP 4 303 298 A1**
(43) Date of publication of application: **10.01.2024**
(21) Application number: 21929220.8
(22) Date of filing: 16.12.2021
(51) Int. Cl.: C12N 5/00, C12M 1/00, C12N 1/00

(54) **CELL FUSION METHOD**

(30) Priority: 01.03.2021 JP 2021031896
(71) Applicant: Daicel Corporation, Osaka 530-0011 (JP); OSAKA UNIVERSITY, Suita-shi Osaka 565-0871 (JP)
(72) Inventor: SAKAGUCHI, Yuko, Tokyo 108-8230 (JP); CHANG, Chin-Yang, Suita-shi, Osaka 565-0871 (JP); MATSUBARA, Yayoi, Tokyo 108-8230 (JP)
(74) Representative: Grünecker Patent- und Rechtsanwälte PartG mbB
(86) International application number: PCT/JP2021/046498
(87) International publication number: WO 2022/185662

(57) **Abstract**

An object of the present disclosure is at least to provide a cell fusion method having a high cell fusion rate. The object is solved by a cell fusion method including pressurizing, by a pressurization portion, a solution containing multiple cells of one or more types and at least one type of chemical cell fusion agent, the solution contained in a container portion.

## Description

### TECHNICAL FIELD

The present disclosure relates to a cell fusion method.

### BACKGROUND ART

Fused cells formed by fusing multiple cells of one or more types are useful as cells having characteristics and functions of each cell before fusion. For example, fused cells are utilized in hybridomas that produce monoclonal antibodies (such as fused cells formed by fusion between B cells and myeloma cancer cells (myeloma)), fused cell vaccines (such as fused cells formed by fusion between dendritic cells and cancer cells from a cancer patient), and regenerative medicine (treatment of diabetes by fusion of pancreatic islet cells and bone marrow cells).

Methods for producing fused cells have been developed, including, for example, an electro cell fusion method (Patent Document 1), Sendai virus methods (Non-Patent Documents 1 and 2), and polyethylene glycol (PEG) methods (Non-Patent Documents 3 and 4).

In the electro cell fusion method, a voltage is applied to cells to perforate cell membranes and cause cell fusion. This method requires adjusting the voltage to an optimal level for perforation according to the cell diameter.

The Sendai virus methods limit targets to animal cells.

The polyethylene glycol (PEG) method is widely used because the use of high molecular weight PEG makes it easy to inhibit the destruction of the cell membrane and because of a higher cell fusion rate and lower cost than other methods. On the other hand, PEG has high cytotoxicity and thus it takes a very long time for a method known in the art to treat PEG, which reduces the survival rate of the cells after treatment. In addition, the method requires complicated operations and many steps that depends on techniques of operators, causing large variations in the results depending on the techniques.

Although the method using PEG is widely used as described above, chemical reagents with cell fusion activity (chemical cell fusion agents) are known in addition to PEG (Non-Patent Document 5).

### PRIOR ART DOCUMENTS

### PATENT DOCUMENT

Patent Document 1: JP 2012-157312 A

### NON-PATENT LITERATURE

Non-Patent Document 1: Nature, 256, 495-497 (1975)
Non-Patent Document 2: J. Immunol., 1; 173 (7): 4297-307 (2004)
Non-Patent Document 3: Somatic Cell Genet., 1 (4): 397-400 (1975)
Non-Patent Document 4: Somatic Cell Genet., 5 (2): 263 to 9 (1979)
Non-Patent Document 5: Maku (Membrane), 6 (5), 310 to 320 (1981)

### SUMMARY OF THE INVENTION

### PROBLEMS TO BE SOLVED BY THE INVENTION

An object of the present disclosure is at least to provide a cell fusion method having a high cell fusion rate.

### MEANS FOR SOLVING THE PROBLEMS

The present inventors have found that the above object can be solved by pressurizing a solution containing multiple cells of one or more types and at least one type of chemical cell fusion agent.

The present disclosure can provide a cell fusion method including:
pressurizing, by a pressurization portion, a solution containing multiple cells of one or more types and at least one type of chemical cell fusion agent, the solution contained in a container portion.

In pressurization by the pressurization portion in the pressurizing in the cell fusion method, time from start of the pressurization until a pressure in the container portion reaches a maximum pressure is preferably 2.0 milliseconds or less, and the maximum pressure is preferably 1.75 MPa or greater and 30.74 MPa or less.

In the cell fusion method, the at least one type of chemical cell fusion agent is preferably polyethylene glycol.

The present disclosure can also provide a cell fusion apparatus including:
a container portion configured to contain a solution containing multiple cells of one or more types and at least one type of chemical cell fusion agent; and
a pressurization portion configured to pressurize the solution contained in the container portion upon actuation.

In the pressurization by the pressurization portion of the apparatus, time from start of the pressurization until a pressure in the container portion reaches a maximum pressure is preferably 2.0 milliseconds or less, and the maximum pressure is preferably 1.75 MPa or greater and 30.74 MPa or less.

### EFFECT OF THE INVENTION

The present disclosure can achieve at least an effect of providing the cell fusion method having a high cell fusion rate. In addition, according to the present disclosure, the method can prevent the occurrence of large variations in results due to techniques of operators and can provide fused cells at a stable ratio.

### BRIEF DESCRIPTION OF THE DRAWINGS

FIG. 1 is a diagram illustrating a schematic configuration of an injection device according to an embodiment.
FIG. 2 is a diagram illustrating a schematic configuration of a falling weight tester according to an embodiment.
FIG. 3 is a diagram illustrating a schematic configuration of an injector assembly to be applied to a falling weight tester according to an embodiment.

### MODE FOR CARRYING OUT THE INVENTION

Note that the configurations, combinations thereof, and the like in the embodiments are each an example, and various additions, omissions, substitutions, and other changes may be made as appropriate without departing from the spirit of the present disclosure. The present disclosure is not limited by the embodiments and is limited only by the claims.

An embodiment of the present disclosure is a cell fusion method including:
pressurizing, by a pressurization portion, a solution containing multiple cells of one or more types and at least one type of chemical cell fusion agent, the solution contained in a container portion.

The container portion in the pressurizing contains a solution containing multiple cells of one or more types and at least one type of chemical cell fusion agent. The structure and material of the container portion are not particularly limited as long as they do not change the quality of the solution contained in the container portion and can withstand the pressure when the solution contained in the container portion is pressurized by the pressurization portion.

The multiple cells of one or more types may be multiple cells of one type or multiple cells of two or more types but are preferably multiple cells of one type, multiple cells of two types, or multiple cells of three types.

When the multiple cells of one or more types are multiple cells of one type, the multiple cells of one type are not particularly limited as long as the cells are fused by the method according to the present embodiment, and the cells may be prokaryotic cells or eukaryotic cells. In addition, the cells may be adherent cells or floating cells. Furthermore, the cells may be cells of established cell lines or primary cultured cells. Moreover, any of these cells may be genetically modified cells.

Examples of the prokaryotic cells include bacterial cells and archaebacterial cells.

Examples of the bacterium include *E. coli, Diplococcus pneumoniae,* lactic acid bacteria, root nodule bacteria, nitrite bacteria, and nitrate bacteria.

Examples of the archaebacterium include extreme halophiles, methanogens, and thermophiles.

Examples of the eukaryotic cells include animal cells, plant cells (including protoplasts), fungal cells, and protist cells.

The animal may be a vertebrate (classified into any of mammals, reptiles, birds, amphibians, and fishes) or may be an invertebrate. Examples of the animal include humans, mice, rats, guinea pigs, hamsters (e.g., such as Chinese hamster), rabbits, donkeys, sheep, goats, alpacas, llamas, chickens, fruit flies, and monkeys (e.g., such as African green monkeys).

The plant may be a seed plant or may be a spore plant (classified into any of pteridophytes, bryophytes, and algae). The seed plant may be an angiosperm or a gymnosperm, and the angiosperm may be a monocotyledon or a dicotyledon.

Examples of the fungus include mushrooms, molds, unicellular yeasts, and zoospores (spores having a flagellum or flagella and moving).

Examples of the protist include eukaryotic algae (e.g., such as brown algae and red algae), fungal organisms having a flagellum or flagella (e.g., oomycetes), slime molds (e.g., such as myxomycetes and cellular slime molds), and protozoans (e.g., such as amebas and *Paramecium caudatum).*

When the multiple cells of one or more types are multiple cells of two or more types, examples of aspects of various cells included in the multiple cells of two or more types include aspects similar to those when the multiple cells of one or more types are multiple cells of one type.

In addition, the combination of the multiple cells of two or more types is not particularly limited as long as the cells are fused by the method according to the present embodiment.

For example, the cells may be multiple cells of two or more types of prokaryotic cells, may be multiple cells of two or more types of eukaryotic cells, or may be cells of one or more types of prokaryotic cells and cells of one or more types of eukaryotic cells.

Furthermore, the cells may be multiple cells of two or more types of adherent cells, may be multiple cells of two or more types of floating cells, or may be cells of one or more types of adherent cells and cells of one or more types of floating cells.

Moreover, the cells may be multiple cells of two or more types of cells of established cell lines, may be multiple cells of two or more types of primary cultured cells, or may be cells of one or more types of cells of established cell lines and cells of one or more types of primary cultured cells.

In addition, the combination of the animals from which the animal cells are derived is not particularly limited either as long as the cells are fused by the method according to the present embodiment. For example, the cells may be multiple cells of two or more types of human-derived cells, may be multiple cells of two or more types of mouse-derived cells, or may be cells of one or more types of human-derived cells and cells of one or more types of mouse-derived cells.

Specific examples include a combination of mouse spleen B cells and mouse myeloma cells (myeloma) (which is used, for example, in the production of monoclonal antibodies), a combination of cancer cells and dendritic cells (which is used, for example, in cancer immunotherapy. The cancer cells and the dendritic cells may be derived from a human, a mouse, or a rat), and a combination of pancreatic islet cells and mesenchymal stem cells (which is used, for example, for treatment of diabetes by insulin secretion. The pancreatic islet cells and the mesenchymal stem cells may be derived from a human or a rat).

In the present disclosure, the chemical cell fusion agent refers to a chemical reagent with cell fusion activity. In the method according to the present embodiment, at least one type of chemical cell fusion agent may be used. The at least one type of chemical cell fusion agent is not particularly limited as long as it is an agent used in cell fusion methods known in the art and the cells are fused by the method according to the present embodiment. In addition, a content of the at least one type of chemical cell fusion agent in the solution is not particularly limited as long as the cells are fused by the method according to the present embodiment but is, for example, 10% (v/v) or greater, and on the other hand, for example, 100% (v/v) or less or 50% (v/v) or less. Furthermore, the upper and lower limits of the content may be a non-contradictory combination of those. Moreover, the multiple cells of one or more types and the at least one type of chemical cell fusion agent can be mixed according to a mixing method known in the art.

Examples of the chemical cell fusion agent include chemical cell fusion agents that are oil-soluble small molecules and chemical cell fusion agents that are water-soluble macromolecules. The chemical cell fusion agent may contain a known auxiliary agent for exhibiting its activity.

Examples of the chemical cell fusion agent that is an oil-soluble small molecule include as described in Non-Patent Document 5:
higher fatty acids (such as decanoic acid, undecanoic acid, undecylenic acid, dodecanoic acid, tridecanoic acid, tetradecanoic acid, myristoleic acid, palmitoleic acid, oleic acid, elaidic acid, linoleic acid, and erucic acid);
higher fatty acid esters (such as methyl undecanoate, methyl dodecanoate, sucrose monolaurate, methyl tridecanoate, methyl tetradecanoate, methyl hexadecanoate, methyl palmitoleate, methyl stearate, methyl oleate, glyceryl monooleate, glyceryl dioleate, ethylene glycol monooleate, and propylene glycol monooleate);
other higher aliphatic derivatives (such as oleylamine, selachyl alcohol, oleyl alcohol, and retinol); and
ionophores (such as A23187, X537A, and valinomycin).

In addition to these, the examples include dimethyl sulfoxide (DMSO), α-tocopherol, 2-(2-methoxy)-ethoxyethyl 9,10-metholeneoctadecanoate, colchicine, amphotericin B, and phospholipase.

As described in Non-Patent Document 5, examples of the chemical cell fusion agent that is a water-soluble macromolecule include polyethylene glycol (PEG) (e.g., PEG with a number average molecular weight of 600 or greater or 1500 or greater and on the other hand 8000 or less or 4000 or less. In addition, the upper and lower limits of the number average molecular weight may be a non-contradictory combination of those. Specific examples include PEG1500, PEG4000, and PEG8000), poly(vinyl alcohol), gelatin, dextran (e.g., molecular weight of 4 × 10⁴ or greater and 2 x 10⁶ or less), dextran sulfate, and poly-L-lysine.

In the pressurizing, the solution is pressurized by the pressurization portion. The pressurization portion is not particularly limited as long as it can pressurize the solution and does not destroy the system, for example, it does not destroy the container portion.

Pressurization by the pressurization portion is not particularly limited as long as the cells are fused by the method according to the present embodiment. For example, for the time from the start of the pressurization until the pressure in the container portion reaches a maximum pressure, any of 0.31 milliseconds, 0.40 milliseconds, 0.48 milliseconds, 0.54 milliseconds, 0.64 milliseconds, 0.80 milliseconds, 1.70 milliseconds, and 2.0 milliseconds may be adopted, and the upper limit or the lower limit may be set by a non-contradictory combination of those.

For example, in view of results of Example 2 and results of Examples 6 to 8 described later, the cell fusion rate is higher when the time is shorter. This is presumed to be because the cells close to each other are attracted to each other at once and this efficiently causes cell fusion. Thus, examples of the upper limit of the time include 2.0 milliseconds or less, 1.70 milliseconds or less, 0.80 milliseconds or less, 0.64 milliseconds or less, and 0.54 milliseconds or less. On the other hand, the lower limit is not particularly limited, but examples include 0.31 milliseconds or greater, 0.40 milliseconds or greater, and 0.48 milliseconds or greater.

In addition, the pressurization by the pressurization portion is not particularly limited as long as the cells are fused by the method according to the present embodiment. For example, any of 1.75 MPa, 2.51 MPa, 5.35 MPa, 6.12 MPa, 6.26 MPa, 11.50 MPa, and 30.74 MPa may be adopted as the maximum pressure, and the upper limit or the lower limit may be set by a non-contradictory combination of those.

Among them, examples of the maximum pressure include 1.75 MPa or greater, 2.51 MPa or greater, 5.35 MPa or greater, 6.26 MPa or greater, and 11.50 MPa or greater because such pressures are presumed to promote the fusion of cell membranes of the cells adhering to each other when the cells are fused. This is in good agreement with a higher cell fusion rate when the maximum pressure is higher, for example, in view of results of Examples 1 to 5 described later. On the other hand, it is presumed that the ratio of formation of a huge cell cluster in which a very large number of cells are bound would increase, and then this would cause a trouble in appropriately culturing the cells and complicate the operation. Thus, the maximum pressure is preferably 30.74 MPa or less.

In addition, the pressurization by the pressurization portion is not particularly limited as long as the cells are fused by the method according to the present embodiment, but from the viewpoint of preventing damage to the cells and increasing the cell survival rate after cell fusion, the pressure is preferably 30.74 MPa or less and more preferably 11.50 MPa or less, and on the other hand, for example, 2.51 MPa or greater and preferably 6.26 MPa or greater. Furthermore, the upper and lower limits of the pressure may be a non-contradictory combination of those.

Here, the pressure means pressure in the container portion. The method for measuring the pressure is not particularly limited, but for example, when the pressure is measured using an injection device described in Examples below, the pressure can be measured, for example, by a method described in JP 2005-21640 A. More specifically, the pressure can be measured by a method described in the section "Method for measuring internal pressure in container portion" below. In addition, also when the pressure is measured using a falling weight tester described in Examples below, the pressure can be measured in the same manner.

The fused cell produced by the method according to the present embodiment is according to the definition of the fused cell in the art and is not limited to a cell produced by fusion of one cell with another cell into a single cell but also includes a cell produced by fusion of three or more cells into a single cell. Under microscopic observation, however, the fused cells may be observed like a cell cluster. Thus, the cell fusion rate in the present disclosure is calculated by a usual calculation method in the art as follows.

When the multiple cells of one or more types are multiple cells of one type, the cell fusion ratio is represented by a ratio of Y to X (i.e., Y/X), where X is the sum of the number of all cell clusters observed with a microscope and the number of cells present as single cells observed with a microscope as in usual culture but not as cell clusters, and Y is "the number of cell clusters formed by fusion of the multiple cells of one type".

Here, "the number of cell clusters formed by fusion of the multiple cells of one type" refers to the number of cell clusters in which two or more cell nuclei can be observed. At this time, the number of cell nuclei can be observed, for example, by microscopic observation or the like by staining a cell cluster with a dye that can stain a nucleus.

When the multiple cells of one or more types are multiple cells of two types, the cell fusion ratio is represented by a ratio of Y to X (i.e., Y/X), where X is the sum of the number of all cell clusters observed with a microscope and the number of cells present as single cells observed with a microscope as in usual culture but not as cell clusters, and Y is "the number of cell clusters formed by fusion of the multiple cells of two types".

Here, "the number of cell clusters formed by fusion of the multiple cells of two types" refers to, when the cells of two types are, for example, cells of cell type A and cells of cell type B, the number of cell clusters formed by fusion of one or more cells of cell type A and one or more cells of cell type B.

At this time, for example, cells of cell type A are stained with dye A and cells of cell type B are stained with dye B in advance, and the number of cell clusters in which both dye A and dye B are detected after cell fusion can be taken as the number of cell clusters.

In addition, the formed cell cluster need not contain one cell for each of the two types, may contain two or more cells for either or both of the two types, and need not contains the same number of cells for the two types. For example, the formed cell cluster may be a cell cluster formed by fusion of one cell of cell type A and one cell of cell type B, a cell cluster formed by fusion of one cell of cell type A and two cells of cell type B, or the like.

When the multiple cells of one or more types are multiple cells of three types, the cell fusion ratio is represented by a ratio of Y to X (i.e., Y/X), where X is the sum of the number of all cell clusters observed with a microscope and the number of cells present as single cells observed with a microscope as in usual culture but not as cell clusters, and Y is "the number of cell clusters formed by fusion of the multiple cells of three types".

Here, "the number of cell clusters formed by fusion of the multiple cells of three types" refers to, when the cells of three types are each, for example, cells of cell type A, cells of cell type B, and cells of cell type C, the number of cell clusters formed by fusion of one or more cells of cell type A, one or more cells of cell type B, and one or more cells of cell type C.

At this time, for example, cells of cell type A are stained with dye A, cells of cell type B are stained with dye B, and cells of cell type C are stained with dye C in advance, and the number of cell clusters in which all of dye A, dye B, and dye C are detected after cell fusion can be taken as the number of cell clusters.

In addition, the formed cell cluster need not contain one cell for each of the three types, may contain two or more cells for any of the three types, and need not contains the same number of cells for the three types. For example, the formed cell cluster may be a cell cluster formed by fusion of one cell of cell type A, one cell of cell type B, and one cell of cell type C, a cell cluster formed by fusion of one cell of cell type A, one cell of cell type B, and two cells of cell type C, or the like.

Also, when the multiple cells of one or more types are multiple cells of four or more types, the cell fusion ratio is similar as described above. That is, the cell fusion ratio is represented by a ratio of Y to X (i.e., Y/X), where X is the sum of the number of all cell clusters observed with a microscope and the number of cells present as single cells observed with a microscope as in usual culture but not as cell clusters, and Y is "the number of cell clusters formed by fusion of the multiple cells of four or more types".

"The number of cell clusters formed by fusion of the multiple cells of four types" can be calculated in the same manner as described above, for example, by assuming cells of cell type D and more in addition to cells of cell type A, cells of cell type B, and cells of cell type C when the multiple cells of one or more types are cells of three types.

In addition, the multiple cells of four types contained in the formed cell cluster can be considered in the same manner as described above.

The fused cells produced by the method according to the present embodiment preferably have a high survival rate. In the present disclosure, the survival rate is calculated by a calculation method commonly used in the art.

For example, when the multiple cells of one or more types are multiple cells of one type, the ratio of the number of viable cells to the total number of cells is calculated by culturing the formed cell cluster for 24 hours under conditions used in usual culture of the multiple cells of one type, then determining the viability using a cell viability determination reagent and if necessary using a device for determining viability, and/or the like.

In addition, when the multiple cells of one or more types are multiple cells of two or more types and the conditions suitable for culturing the cells vary depending on the types of cells, the cells are cultured under conditions suitable for culturing the type of cells most vulnerable to environmental changes among the multiple cells of two or more types as performed in the art. In the culture, a necessary component may be appropriately added. Then, the ratio of the number of viable cells to the total number of cells is calculated by determining the viability using a cell viability determination reagent and if necessary using a device for determining viability, and/or the like.

When the total number of cells is 4 x 10⁶ or greater, the survival rate is preferably 32.75% or greater, more preferably 41.25% or greater, and even more preferably 48.25% or greater. On the other hand, the upper limit is preferably higher but is, for example, 100% or less or 54.88% or less. In addition, the upper and lower limits of the survival rate may be a non-contradictory combination of those.

Another embodiment of the present disclosure is a cell fusion apparatus including:
a container portion configured to contain a solution containing multiple cells of one or more types and at least one type of chemical cell fusion agent; and
a pressurization portion configured to pressurize the solution contained in the container portion upon actuation.

As already described, the structure and material of the container portion are not particularly limited as long as they do not change the quality of the solution contained in the container portion and can withstand the pressure when the solution contained in the container portion is pressurized by the pressurization portion.

The apparatus according to the present embodiment may include a drive portion for allowing the pressurization portion to pressurize the solution contained in the container portion. The structure and material of the drive portion are not particularly limited. The pressurization may, for example, be provided by the pressure generated when the pressure of compressed gas is released or by the pressure generated by the combustion of an explosive ignited by an ignition apparatus. Alternatively, the pressurization may be provided by the pressure utilizing electrical energy of a piezoelectric element or the like or mechanical energy of a spring or the like as the pressurization energy, and by the pressure utilizing the pressurization energy generated by appropriately combining these forms of energy.

When an aspect in which the pressure generated by the combustion of an explosive ignited by an ignition apparatus is employed as the pressurization, examples of the explosives include any one of an explosive containing zirconium and potassium perchlorate (ZPP), an explosive containing titanium hydride and potassium perchlorate (THPP), an explosive containing titanium and potassium perchlorate (TiPP), an explosive containing aluminum and potassium perchlorate (APP), an explosive containing aluminum and bismuth oxide (ABO), an explosive containing aluminum and molybdenum oxide (AMO), an explosive containing aluminum and copper oxide (ACO), an explosive containing aluminum and iron oxide (AFO), or an explosive composed of a combination of a plurality of these explosives. As characteristics of these explosives, the combustion product is gas at a high temperature but does not include a gas component at a room temperature, hence the combustion product is condensed immediately after the ignition. In the pressurization process of the solution, this enables the temperature and pressure of the combustion product generated by the combustion of an ignition charge to shift, under the pressurization, to the vicinity of normal temperature and pressure in a short period of time after the pressure applied to the solution reaches the first peak injection force.

Examples of the apparatus according to the present embodiment include an injection device. Details of the injection device will be described below.

In the injection device as an example of the device according to the present embodiment, the multiple cells of one or more types and the at least one type of chemical cell fusion agent are not initially contained in the container portion. For example, in a state where a syringe portion is detached from the injection device and in a state where a sealing member described later is detached from the syringe portion, the multiple cells of one or more types and the at least one type of chemical cell fusion agent are injected into the syringe portion from the upper open end side of the syringe portion (an open end located on the side opposite an injection port in the axial direction). An end portion located on the side opposite the injection port in the axial direction in the syringe portion is formed as an open end, and the open end is open to the outside in a state where a plunger is not inserted into the syringe portion. After the multiple cells of one or more types and the at least one type of chemical cell fusion agent are injected into the syringe portion, the plunger is slightly inserted into the syringe portion from the upper open end side without leaking the cells and the at least one type of chemical cell fusion agent from the upper open end of the syringe portion and a nozzle, and this forms the container portion in the syringe portion accordingly. This eliminates the leakage of the multiple cells of one or more types and the at least one type of chemical cell fusion agent contained in the container portion in the syringe portion from the container portion even when the posture of the syringe portion is changed to direct the upper open end downward. Then, for example, the posture of the syringe portion is changed to direct the injection port upward, and the plunger is slid along an inner wall surface extending in the axial direction of the syringe portion to expel gas in the container portion from the injection port through the nozzle to the outside of the container portion. The configuration thus requiring a filling operation into the container portion is employed, and this enables the desired multiple cells of one or more types and the desired at least one type of chemical cell fusion agent to be contained. For this purpose, in the injection device, the syringe portion is configured to be attachable and detachable.

In addition, during operation of the injection device, the injection port at the tip of the nozzle is sealed to prevent the injection of the multiple cells of one or more types and the at least one type of chemical cell fusion agent. The sealing member and the sealing method are not particularly limited as long as they prevent injection of the multiple cells of one or more types and the at least one type of chemical cell fusion agent.

Hereinafter, an injector 1 (needle-free injector with a processed sealable tip side) will be described as an example of the injection device with reference to the drawing. It should be noted that the respective configurations and the combinations thereof in the respective embodiments are only examples, and the configurations may be added, omitted, substituted, or otherwise modified as appropriate within a scope that does not depart from the spirit of the present disclosure. The present disclosure is not limited by the embodiments and is limited only by the claims. This applies to the examples described later. Note that as terms indicating a relative positional relationship in a longitudinal direction of the injector 1, "tip side" and "base end side" are used. The "tip side" represents the position which is deviated toward the tip of the injector 1 as described later on, i.e., deviated toward the injection port 31a. The "base end side" represents the direction opposite to the "tip side" in the longitudinal direction of the injector 1, i.e., the direction toward the drive portion 7 side. In addition, the present example is an example of pressurizing the container portion for containing the multiple cells of one or more types and the at least one type of chemical cell fusion agent using combustion energy of an explosive ignited by an ignition apparatus, but the present embodiment is not limited to this.

### (Configuration of Injector 1)

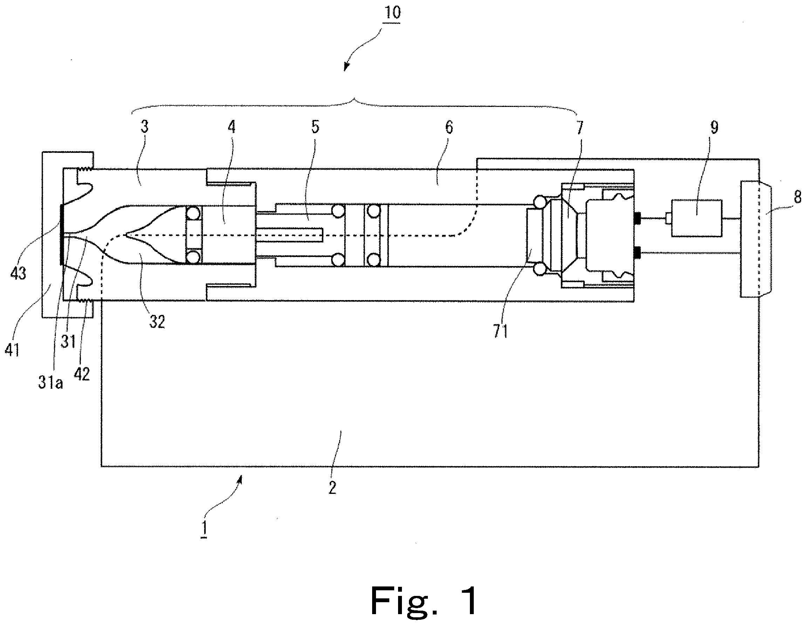

FIG. 1 is a cross-sectional view of the injector 1, taken along the longitudinal direction thereof, illustrating a schematic configuration of the injector 1. The injector 1 is formed by attaching an injector assembly 10 to a housing (injector housing) 2. The injector assembly 10 includes a subassembly including a syringe portion 3 and a plunger 4 and a subassembly including an injector body 6, a piston 5, and a drive portion 7, and the subassemblies are integrally assembled.

As described above, the injector assembly 10 is configured to be detachable from and attachable to the housing 2. A container portion 32 formed between the syringe portion 3 and the plunger 4 included in the injector assembly 10 is filled with the multiple cells of one or more types and the at least one type of chemical cell fusion agent. The injector assembly 10 is a unit to be disposed each time cell fusion is performed. In addition, performing cell fusion in a sterile environment facilitates the production of fused cells in sterile conditions. A battery 9 that supplies power to an igniter 71 included in the drive portion 7 of the injector assembly 10 is included on the housing 2 side. The power supply from the battery 9 is performed between an electrode on the housing 2 side and an electrode on the drive portion 7 side of the injector assembly 10 through wiring, when a user performs an operation of pressing a button 8 provided to the housing 2. The electrode on the housing 2 side and the electrode on the drive portion 7 side of the injector assembly 10 have shapes and positions designed to come into contact with each other automatically when the injector assembly 10 is attached to the housing 2. Further, the housing 2 is a unit that can be repeatedly used as long as power that can be applied to the drive portion 7 is left in the battery 9. When the battery 9 runs out of power in the housing 2, the housing 2 may be continued to be used with only the battery 9 exchanged. In addition, the injection port 31a at the tip of a nozzle 31 is sealed by a sealing portion 43 to prevent injection of the multiple cells of one or more types and the at least one type of chemical cell fusion agent. The sealing portion 43 is fixed to a cap 41. The cap 41 is fixed to the syringe portion 3 via a fixing portion 42.

Next, the details of the injector assembly 10 will be described. First, a description is given on the subassembly including the syringe portion 3 and the plunger 4. In the syringe portion 3, the container portion 32 is formed as a space that can contain the multiple cells of one or more types and the at least one type of chemical cell fusion agent. More specifically, as illustrated in FIG. 1, the plunger 4 is disposed to be slidable along an inner wall surface extending in the axial direction of the syringe portion 3, and the container portion 32 is defined by the inner wall surface of the syringe portion 3 and the plunger 4. The syringe portion 3 includes a nozzle portion 31 having the injection port 31a formed on the tip side. In the example illustrated in FIG. 1, the plunger 4 has a contour on the tip side shaped to substantially match the contour of the inner wall surface of the nozzle portion 31.

Furthermore, the syringe portion 3 includes the fixing portion 42 for fixing the cap 41. The cap 41 is fixed to the fixing portion 42. The cap 41 has the sealing portion 43 for sealing the injection port 31a. The injection port 31a of the nozzle portion 31 is sealed by the sealing portion 43 in a state where the cap 41 is fixed to the fixing portion 42 of the syringe portion 3. In this state, the container portion 32 in the syringe portion 3 is sealed in a hermetically sealed state. The cap 41 can be detachably fixed to the fixing portion 42 of the syringe portion 3. The nozzle portion 31 in the syringe portion 3 has a flow path that communicates with the injection port 31a and the container portion 32 as illustrated in FIG. 1, and the flow path has a flow path cross sectional area gradually decreasing from the container portion 32 side toward the injection port 31a side.

Next, the subassembly including the injector body 6, the piston 5, and the drive portion 7 will be described. The piston 5 is made of metal, for example, and is configured to be pressurized by a combustion product (combustion gas) generated by the igniter 71 of the drive portion 7 and to slide in a through hole formed in the injector body 6. The injector body 6 is a substantially cylindrical member, and the piston 5 is contained therein to be slidable along the inner wall surface extending in the axial direction thereof. The piston 5 may be formed of a resin, and in such a case, metal may be used together for a part to which heat resistance and pressure resistance are required. As illustrated in FIG. 1, the piston 5 is integrally coupled with the plunger 4. In the present embodiment, a "pressurization portion" is constituted including the plunger 4 and the piston 5.

Next, the drive portion 7 will be described. As illustrated in FIG. 1, the drive portion 7 is fixed to a base end side with respect to the through hole in the injector body 6. The drive portion 7 includes the igniter 71, which is an electric igniter. The igniter 71 is disposed to face the interior of the through hole in the injector body 6, and contains an ignition charge therein. As the ignition charge, various types of explosives can be employed as described above. In addition, the ignition charge can be contained in an explosive cup formed by an appropriate thin metal, for example.

Next, how the injector 1 having the configuration described above is operated will be described. As described above, the syringe portion 3 is configured to be attachable and detachable. In a state where the syringe portion 3 is detached from the injection device 1 and in a state where the cap 41 is detached from the fixing portion 42 of the syringe portion 3, the multiple cells of one or more types and the at least one type of chemical cell fusion agent are injected into the syringe portion 3, for example, from the upper open end side of the syringe portion 3 (an open end located on the side opposite the injection port in the axial direction). The plunger 4 is slightly inserted into the syringe portion 3 from the upper open end side without leaking the cells and the at least one type of chemical cell fusion agent from the upper open end of the syringe portion 3 and the nozzle. The posture of the syringe portion 3 is changed to direct the injection port 31a upward, and the plunger 4 is slid along an inner wall surface extending in the axial direction of the syringe portion 3 to expel gas in the container portion 32 from the injection port 31a through the nozzle portion 31 to the outside of the container portion 32.

This enables the desired multiple cells of one or more types and the desired at least one type of chemical cell fusion agent to be contained in the container portion 32. At this time, the multiple cells of one or more types and the at least one type of chemical cell fusion agent are preferably contained in the container portion 32 in a state of being mixed in advance as in the related art. However, the aspect is not particularly limited as long as the multiple cells of one or more types and the at least one type of chemical cell fusion agent can be finally contained in the container portion 32 in a mixed state as in the related art.

In addition, when the solution containing the multiple cells of one or more types and the at least one type of chemical cell fusion agent are pressurized by the pressurization portion, gas (such as air) is preferably not contained in the container portion 32. An example of the procedure for this includes removing air from the container portion 32 through the injection port 31a after the multiple cells of one or more types and the at least one type of chemical cell fusion agent are contained in the container portion 32 as described above.

Next, the cap 41 is attached to the fixing portion 42 of the syringe portion 3. As a result, the injection port 31a of the nozzle portion 31 is sealed by the sealing portion 43, and thus the container portion 32 is hermetically sealed.

In this state, when the user performs an operation of pressing the button 8 provided to the housing 2, for example, this serves as a trigger to supply the actuation power from the battery 9 to the igniter 71 of the drive portion 7, and thus the igniter 71 is activated. Upon actuation of the igniter 71, the ignition charge is ignited and thus combusted, and combustion products (flame, combustion gas, and the like) are generated. As a result, the explosive cup of the igniter 71 is ruptured, for example, and the combustion gas of the ignition charge is released into the through hole in the injector body 6. Thus, the pressure in the through hole of the injector body 6 rapidly increases, and the piston 5 is pressed toward the tip side of the injector body 6. As a result, the piston 5 slides along the inner wall surface of the through hole in the injector body 6 toward the tip side. As described above, because the plunger 4 is coupled integrally with the piston 5, the plunger 4 also slides along the inner wall surface of the syringe portion 3 in conjunction with the piston 5. That is, the plunger 4 is pushed toward the nozzle portion 31 located on the tip side of the syringe portion 3, and this reduces the volume of the container portion 32 containing the multiple cells of one or more types and the at least one type of chemical cell fusion agent and rapidly pressurizes the solution.

As described above, upon actuation of the igniter 71 in the drive portion 7, the plunger 4 is pushed through the piston 5 by the combustion energy of the ignition charge, and this rapidly pressurizes the liquid contained in the container portion 32 in a hermetically sealed state. Here, in the injector 1, the type and dose of the ignition charge, and any other optional parameter are adjusted to actuate the drive portion 7 (igniter 71) to allow the pressurization portion to pressurize the solution contained in the container portion. As a result, cell fusion is suitably performed. After fused cells are thus produced, for example, the injector assembly 10 is removed from the housing 2, and then the cap 41 is removed from the syringe portion 3. Then, the syringe portion 3 into which the plunger 4 is inserted may be removed, the plunger 4 may be further removed from the syringe portion 3, and the content containing the fused cells contained in the container portion 32 may be taken out, for example, with a pipette and collected in an appropriate container.

As described above, with the injector 1 as an example of the apparatus according to the present embodiment, cell fusion can be easily performed without requiring a large apparatus and a high level of skill of the engineer to handle it. Furthermore, with the injector 1, the injector assembly 10 is detachably attached to the housing 2, and the injector assembly 10 can be configured as a disposable unit. Thus, a used injector assembly 10 is disposed of after production of fused cells. This eliminates the need for cleaning the used injector assembly 10 every time cell fusion is performed, which can reduce the time and labor of the user and provide a cell fusion apparatus having excellent usability.

Examples of the cell fusion apparatus according to the present embodiment also include a falling weight tester. Hereinafter, the falling weight tester will be described with reference to the drawing.

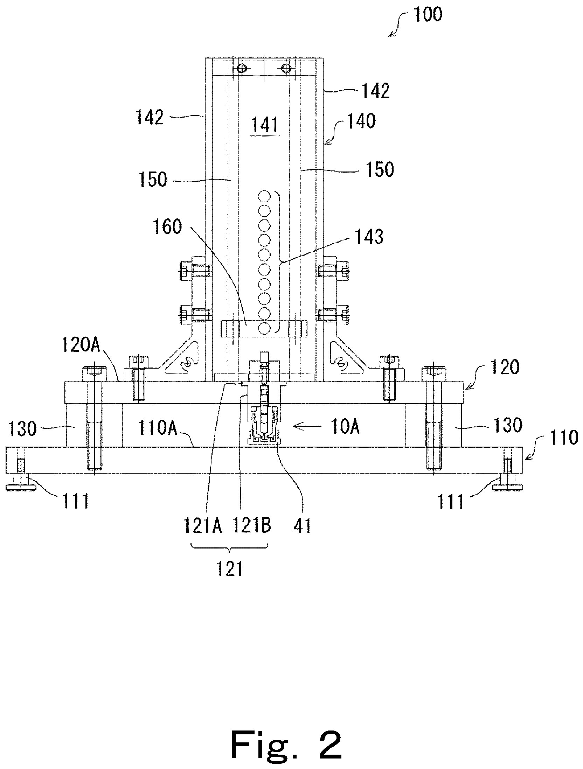

FIG. 2 is a diagram illustrating a schematic configuration of a falling weight tester 100. The falling weight tester 100 includes a bed 110, a support plate 120, support columns 130 supporting the support plate 120, a frame 140 standing upward from the support plate 120, guide rods 150, a weight 160, and an injector assembly 10A to be set in the support plate 120.

The bed 110 is a base to be placed on an appropriate placement surface, such as a floor or table, and has a plurality of leg portions 111 for placing the bed 110 on the placement surface. For example, the bed 110 is a planar rectangular-shaped steel plate, and the leg portions 111 may be provided at its four corners.

As illustrated in FIG. 2, the support plate 120 is a plate member that is fixed to the bed 110 through the support columns 130 standing from an upper surface 110A of the bed 110 and is disposed to face the bed 110 in parallel with a gap between them. For example, the support plate 120 is a planar rectangular-shaped steel plate. For example, at a planar center portion of the support plate 120, a holding hole 121 for detachably holding the injector assembly 10A is provided to penetrate the support plate 120 in the thickness direction. The holding hole 121 includes a holding recessed portion 121A that is open to an upper surface 120A side of the support plate 120 and a small-diameter hole portion 121B that is open to a lower surface 120B side of the support plate 120 and has a smaller diameter than the holding recessed portion 121A. The small-diameter hole portion 121B is connected to the lower side of the holding recessed portion 121A.

As illustrated in FIG. 2, a pair of cylindrical guide rods 150 stands on the upper surface 120A of the support plate 120. The guide rods 150 are each a rod member for guiding a falling direction of the weight 160 when the weight 160 is dropped from a predetermined height. In the weight 160, a pair of insertion holes (not illustrated) for inserting the pair of guide rods 150 is formed to penetrate from the upper surface 160A to the lower surface 160B. Each of the guide rods 150 is inserted into the corresponding insertion hole of the weight 160, and this enables the weight 160 to fall along the guide rods 150. In the present embodiment, the guide rods 150 stand in the direction perpendicular to the upper surface 120A of the support plate 120.

In the present specification, dropping the weight 160 from a predetermined height refers to dropping the weight 160 vertically downward from the predetermined height in the atmosphere without imparting an initial velocity.

In addition, as illustrated in FIG. 2, the frame 140 stands on the upper surface 120A of the support plate 120. The frame 140 is a frame member that has a back frame 141 and a pair of side frames 142 connected to both side edges of the back frame 141 and has a substantially C-shaped cross section. In the example illustrated in FIG. 2, the guide rods 150 are disposed in an inner space surrounded by the frame 140. The back frame 141 is provided with a plurality of insertion holes 143 for detachably inserting pins (not illustrated) for positioning the weight 160 at a predetermined height. For example, the insertion holes 143 are formed at regular intervals (e.g., 10-mm intervals) in the height direction of the back frame 141. The pin can be selectively inserted into any of the insertion holes 143. The pin is inserted into a desired insertion hole 143 from the back surface side of the back frame 141, and the pin is inserted into a fixing hole formed in the back surface of the weight 160, and this can fix the weight 160 in a state of being positioned at a desired height. In addition, from this state, the pin is pulled out to release the fixation of the weight 160, and this results in enabling the weight 160 to fall downward along the guide rods 150. In the present embodiment, a plurality of weights 160 with different weights (e.g., such as 30 g, 40 g, 50 g, and 300 g) can be prepared.

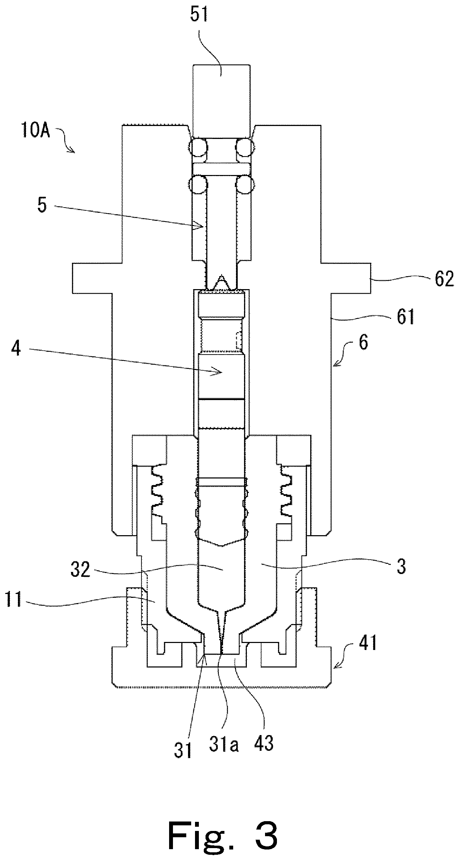

Next, the injector assembly 10A that can be held in the holding hole 121 of the support plate 120 will be described. FIG. 3 is a diagram illustrating a schematic configuration of the injector assembly 10A to be applied to the falling weight tester 100. The injector assembly 10A includes a syringe portion 3, a plunger 4, a piston 5, an injector body 6, a holder 11, and the like. The injector body 6 has, for example, a substantially cylindrical shape and has a through hole formed in the axial direction. In the through hole of the injector body 6, the piston 5 and the plunger 4 are movably disposed along the through hole.

The holder 11 is a member attached to the tip side of the injector body 6 and holding the syringe portion 3 in its inside. In the syringe portion 3, the plunger 4 is slidably disposed along an inner wall surface extending in the axial direction of the syringe portion 3, and the container portion 32 is defined by the inner wall surface of the syringe portion 3 and the plunger 4. In addition, the syringe portion 3 has a nozzle portion 31 in which the injection port 31a is formed on the tip side. In the example illustrated in FIG. 3, the contour on the tip side of the plunger 4 is shaped to substantially match the contour of the inner wall surface of the nozzle portion 31. Similarly to the injector assembly 10 described in FIG. 1, the container portion 32 can contain the multiple cells of one or more types and the at least one type of chemical cell fusion agent. The holder 11 and the syringe portion 3 are attachable to and detachable from the injector body 6.

A cap 41 can be detachably attached to the tip side of the holder 11. In a state where the cap 41 is attached to the holder 11, the injection port 31a of the nozzle portion 31 is sealed by a sealing portion 43 of the cap 41, and as a result, the container portion 32 in the syringe portion 3 is in a hermetically sealed state. The nozzle portion 31 in the syringe portion 3 has a flow path that communicates with the injection port 31a and the container portion 32 as illustrated in FIG. 3, and the flow path cross sectional area of the flow path gradually decreases from the container portion 32 side toward the injection port 31a side.

The piston 5 is, for example, made of metal and is configured to be slidable in a through hole formed inside the injector body 6. The front end portion of the piston 5 is connected to the rear end portion of the plunger 4. In addition, a weight receiving portion 51 is formed at the rear end portion of the piston 5. The weight receiving portion 51 of the piston 5 is exposed to the outside to protrude from the rear end of the injector body 6 through the through hole of the injector body 6. A flange 62 with a circular flange shape is formed on the outer circumferential surface 61 of the injector body 6. The outer diameter at the outer circumferential surface 61 of the outer circumferential surface 61 of the injector body 6 is smaller than the small-diameter hole portion 121B of the support plate 120. In addition, the flange 62 in the injector body 6 has a diameter slightly smaller than that of the holding recessed portion 121A and larger than that of the small-diameter hole portion 121B in the support plate 120. The holding recessed portion 121A in the support plate 120 is formed as a recessed portion for supporting the flange 62 of the injector body 6. The injector assembly 10A configured as described above to be included in the falling weight tester 100 is different from the injector assembly 10 described in FIG. 1 in that the injector assembly 10A does not include the drive portion 7 including the igniter 71.

Next, how the falling weight tester 100 including the injector assembly 10A is operated will be described. Before the injector assembly 10A is set on the support plate 120, desired multiple cells of one or more types and desired at least one type of chemical cell fusion agent are contained in the container portion 32 of the injector assembly 10A. These solutions to be contained may be contained in the container portion 32 in the same procedure as in the case of using the injector 1 described above. That is, in a state where the syringe portion 3 is detached from the injector body 6 and in a state where the cap 41 is detached from the holder 11, the solutions to be contained described above (the multiple cells of one or more types and the at least one type of chemical cell fusion agent) are injected into the syringe portion 3, for example, from the upper open end side of the syringe portion 3 (an open end located on the side opposite the injection port in the axial direction). The plunger 4 is slightly inserted into the syringe portion 3 from the upper open end side without leaking these solutions from the upper open end of the syringe portion 3 and the nozzle. Then, the posture of the syringe portion 3 is changed to direct the injection port 31a upward, and the plunger 4 is slid along the inner wall surface extending in the axial direction of the syringe portion 3 to expel gas in the container portion 32 from the injection port 31a through the nozzle portion 31 to the outside of the container portion 32. This enables the desired multiple cells of one or more types and the desired at least one type of chemical cell fusion agent to be contained in the container portion 32.

Next, the cap 41 is attached to the holder 11 in the injector assembly 10A to seal the injection port 31a of the nozzle portion 31, thus hermetically sealing the container portion 32. The injector assembly 10A with the container portion 32 thus hermetically sealed is placed on the support plate 120 as illustrated in FIG. 2. That is, in a state where the cap 41 of the injector assembly 10 is directed downward, the injector assembly 10A is inserted into the holding hole 121 of the support plate 120, and the flange 62 of the injector body 6 is fitted into the holding recessed portion 121A, thereby completing the placement of the injector assembly 10A on the support plate 120. At this time, a slight gap is formed between the cap 41 and the upper surface 110A of the bed 110 as illustrated in FIG. 2. That is, the injector assembly 10A is supported and fixed to the support plate 120 in a state of being separated from the bed 110.

The falling weight tester 100 pressurizes the solution contained in the container portion 32 by utilizing energy generated when the weight 160 falling from a predetermined height collides with the weight receiving portion 51 of the piston 5 in the injector assembly 10A. The weight 160 is set at a predetermined height using a desired insertion hole 143 provided in the frame 140 and the pin. In pressurizing the solution contained in the container portion 32 of the injector assembly 10A, the pin fixing the weight 160 is pulled out from the insertion hole 143. As a result, the weight 160 falls downward along the guide rod 150. Here, the bed 110 is adjusted to be in a horizontal posture, and this allows the guide rod 150 to be in a state of extending along the vertical direction. As a result, the weight 160 falls vertically downward. The weight receiving portion 51 of the piston 5 is positioned below the weight 160, the weight 160 falling from the predetermined height collides with the weight receiving portion 51 of the piston 5. As a result of thus the collision energy of the weight 160 being imparted to the piston 5, the piston 5 slides toward the tip side along the inner wall surface of the through hole in the injector body 6. This also allows the plunger 4 connected to the piston 5 to slide along the inner wall surface of the syringe portion 3, and the plunger 4 is pushed toward the nozzle portion 31. This reduces the volume of the container portion 32, and the solution contained in the container portion 32 is rapidly pressurized. As a result, cell fusion is suitably performed. In the falling weight tester 100, the "pressurization portion" is constituted including the plunger 4 and the piston 5 of the injector assembly 10A.

After the fused cells are thus produced as described above, for example, the weight 160 is removed, and the injector assembly 10A is removed from the support plate 120. Then, the cap 41 of the injector assembly 10A is removed, and the syringe portion 3 into which the plunger 4 is inserted is removed from the injector body 6. Furthermore, the content containing the fused cells contained in the container portion 32 of the syringe portion 3 from which the plunger 4 is removed may be taken out, for example, with a pipette and collected in an appropriate container.

The time from the start of the pressurization until the pressure in the container portion reaches a maximum pressure can be extended by increasing the weight of the weight, and the height when the weight is dropped does not affect the time. Thus, the time can be set appropriately, for example, by preparing weights of various weights and dropping them as a preliminary test to clarify a relationship between the weight of the weight and the time. In addition, the time can also be extended by placing a cushioning material (e.g., such as sponge rubber) on the piston. Thus, the time can be set appropriately after performing a preliminary test in the same manner as described above.

Furthermore, the maximum pressure can be increased by increasing the load (the weight of the weight x the height from which the weight is dropped). Thus, the maximum pressure can be set appropriately, for example, by preparing weights of various weights and dropping them from various heights as a preliminary test to clarify a relationship between the weight of the weight, the height from which the weight is dropped, and the maximum pressure.

As described above, the time from the start of the pressurization until the pressure in the container portion reaches a maximum pressure and the maximum pressure can be appropriately set independently of each other.

### EXAMPLES

Examples will be described below, but none of the examples shall be construed as limiting.

### <Method for Measuring Internal Pressure in Container Portion>

In Examples 1 to 4, the injection device illustrated in FIG. 1 was used as a cell fusion apparatus, and mouse spleen cells and NS-1 cells were fused in the container portion of the injection device.

The time from the start of the pressurization until the pressure reaches a maximum pressure and the maximum pressure were measured as follows. According to the measurement method described in JP 2005-21640 A, an injection force was measured by a method in which the injection force was applied in a dispersed manner to a diaphragm of a load cell disposed downstream of a nozzle, and output from the load cell was collected by a data collection device through a detection amplifier and was stored as an injection force (N) per time. The injection pressure was calculated by dividing the injection force thus measured by the area of the injection port 31a of the injection device. The volume of the container portion was 100 µL, the container portion was filled with Milli-Q Water (Millipore Corporation), air was removed, and the measurement was performed.

The injection pressure measured as described above was confirmed to be approximately equivalent to the internal pressure of the container portion determined by the measurement method described in WO 2020/116353. In addition, replacement of the Milli-Q Water in the container portion with a solution used in examples described later does not affect the time from the start of the pressurization until the pressure reaches a maximum pressure and the maximum pressure.

In Examples 5 to 8, the falling weight tester illustrated in FIG. 2 was used as the cell fusion apparatus. The time from the start of the pressurization until the pressure reaches a maximum pressure and the maximum pressure were measured in the same manner as described above using a load cell.

### <Method for Mixing Cells>

### (Preparation of Mouse Spleen Cells)

Mouse spleen cells were collected from BALB/c mice (female, from 6 to 8 weeks old or the like). That is, after the mice were euthanized, the spleens were collected and washed with PBS (Nacalai Tesque). Homogenized spleen cells were filtered with a 70 µm Cell Strainer (Falcon), and then collected in a 15-mL centrifuge tube (Violamo). To lyse red blood cells, ACK Lysing Buffer (GIBCO) in an amount 1.5 times the organ weight was added, and the centrifuge tube was allowed to stand at room temperature for 5 minutes. For washing, 45 mL of PBS was added, the mixture was centrifuged (700 x g for 5 minutes), and the supernatant was removed. Brilliant Violet 421 anti-mouse CD45 Antibody (30-F11, BIO LEGEND) in an amount of 20 µL was added per mL of a cell suspension prepared by adjusting to 1 × 10⁸ cells/mL with PBS, and fluorescent staining with the antibody was performed at 4°C for 15 minutes. The cells were washed twice with PBS in an amount ten times that of the staining solution and then adjusted to 3 × 10⁷ cells/mL with PBS.

### (Preparation of NS-1 Cells)

NS-1 cells (purchased from JCRB Cell Bank, Cell Accession No. JCRB9107) were prepared by thawing a cell stock previously cryopreserved by passage. The cells were adjusted to 1 × 10⁶ cells/mL with PBS, 5 µL/mL of DIR (5 mg/mL in DMSO, Invitrogen) was added, and fluorescent staining was performed in a CO₂ incubator (37°C, 95% CO₂) for 10 minutes. The cells were washed three times with PBS in an amount five times that of the staining solution and then adjusted to 1 × 10⁷ cells/mL with PBS.

### (Mixing of Cells)

The stained mouse spleen cells and NS-1 cells were mixed at a ratio of 3:1. That is, 100 µL each of the mouse spleen cells and NS-1 cells was transferred to the same 1.5 mL tube with a micropipette and centrifuged (700 x g for 5 minutes). The supernatant was removed as much as possible, and a pellet containing 4 × 10⁶ cells as the total number of cells was prepared. The 1.5-mL tube was gently tapped to loosen the pellet, the cells were mixed, and a cell mixed liquid was prepared.

### <Method for Measuring Cell Fusion Rate>

Y, the "number of fused cells", was determined by performing the flow cytometry on the fused cell liquid produced by the cell fusion in such a manner that 5000 cells were flowed in a flow cytometer (Beckman) with PB450 and APCA750 filters, and cells in which both fluorescences were detected were counted as fused cells.

That is, the cell fusion ratio was represented by a ratio of Y to 5000 cells (i.e., Y/5000), where 5000 was X, the sum of the number of all cell clusters and the number of cells present as single cells as in usual culture but not as cell clusters, and Y was the number of fused cells.

### <Method for Measuring Cell Survival Rate>

The fused cell liquid was seeded in a 6-well plate (Violamo) and cultured in a CO₂ incubator (37°C, 95% CO₂) for 24 hours. Some of the cells were stained with trypan blue (Nacalai Tesque), the numbers of stained cells and unstained cells were counted with a TC20 fully automated cell counter (Bio-Rad), and the cell survival rate was calculated. The cell survival rate was defined as a ratio of the number of viable cells to the total number of cells measured.

### (Example 1)

The pellet produced according to "Method for Mixing Cells" described above was subjected to the following operations. The tip of a pipet was placed at the time of pelleting, and the pellet and PEG1500 were mixed by slowly performing the following at the same time: an operation of ejecting 100 µL of PEG1500 (polyethylene glycol with a number average molecular weight of 1500, Roche) like gently placing it; and an operation like drawing a circle with the tip of the pipet. Then, the mixture was slowly pipetted six to seven times. This cell mixed liquid was filled from the upper open end side of the syringe portion, and the plunger was slightly inserted into the syringe portion from the upper open end side without leaking these from the upper open end of the syringe portion and the nozzle. The posture of the syringe portion was changed to direct the injection port upward, and the plunger was slid along the inner wall surface extending in the axial direction of the syringe portion to expel air in the container portion from the injection port through the nozzle portion to the outside of the container portion. Here, the injection device was used as the cell fusion apparatus and was the injection device illustrated in FIG. 1. In the present example, the injection device was set to a condition of having 15 mg of ZPP, and the ignition operation was performed in a state where the inside of the container portion was hermetically sealed by securely mounting the cap on the nozzle side of the container portion. This was expected to enable fusion of the mouse spleen cells and the NS-1 cells. In this condition, the time from the start of the pressurization until the pressure reaches a maximum pressure was 0.31 milliseconds, and the maximum pressure was 2.51 MPa. Then, the injector assembly was removed from the housing, the cap was removed from the syringe portion, the syringe portion into which the plunger had been inserted was removed, the plunger was further removed from there, and the contents of the syringe portion were drawn up with a Pipetman. The contents were mixed by slowly pipetting in a 1.5-mL tube together with 1 mL of RPMI 1640 (Nacalai Tesque) pre-warmed to 37°C. The mixture was centrifuged (500 x g for 10 minutes), and the cells were washed. After removing the supernatant, the cells were suspended in PBS, and a fused cell liquid was formed.

Then, the cell fusion rate and the cell survival rate were each measured.

### (Examples 2 to 4)

The procedure was performed in the same manner as in Example 1 except that the amount of ZPP as the explosive was changed.

### (Example 5)

The pellet produced according to "Method for Mixing Cells" described above was subjected to the following operations. The tip of a pipet was placed at the time of pelleting, and the pellet and PEG1500 were mixed by slowly performing the following at the same time: an operation of ejecting 100 µL of PEG1500 (polyethylene glycol with a number average molecular weight of 1500, Roche) like gently placing it; and an operation like drawing a circle with the tip of the pipet. Then, the mixture was slowly pipetted six to seven times. This cell mixed liquid was filled from the upper open end side of the syringe portion, and the plunger was slightly inserted into the syringe portion from the upper open end side without leaking these from the upper open end of the syringe portion and the nozzle. The posture of the syringe portion was changed to direct the injection port upward, and the plunger was slid along the inner wall surface extending in the axial direction of the syringe portion to expel air in the container portion from the injection port through the nozzle portion to the outside of the container portion. Next, the inside of the container portion was pressurized using the falling weight tester illustrated in FIG. 2. In the present example, the falling weight tester was set to conditions of dropping a 50 g weight from a position at a height of 30 mm, and the weight was dropped in a state where the inside of the container portion was hermetically sealed by securely mounting the cap on the nozzle side of the container portion. This was expected to enable fusion of the mouse spleen cells and the NS-1 cells. In this condition, the time from the start of the pressurization until the pressure reaches a maximum pressure was 0.64 milliseconds, and the maximum pressure was 1.75 MPa. Then, the injector assembly was removed from the falling weight tester, the cap was removed from the syringe portion, the syringe portion into which the plunger had been inserted was removed, the plunger was further removed from there, and the contents of the syringe portion were drawn up with a Pipetman. The contents were mixed by slowly pipetting in a 1.5-mL tube together with 1 mL of RPMI 1640 (Nacalai Tesque) pre-warmed to 37°C. The mixture was centrifuged (500 x g for 10 minutes), and the cells were washed. After removing the supernatant, the cells were suspended in PBS, and a fused cell liquid was formed.

Then, the cell fusion rate was measured.

### (Example 6)

The procedure was performed in the same manner as in Example 5 except that a 300 g weight was dropped from a position at a height of 20 mm and an EPDM sponge rubber (Wakisangyo, EPT-01S) was placed on the piston. The EPDM sponge rubber (Wakisangyo, EPT-01S) on the piston was placed to increase the time from the start of the pressurization until the pressure in the container portion reaches a maximum pressure.

### (Example 7)

The procedure was performed in the same manner as in Example 5 except that a 300 g weight was dropped from a position at a height of 10 mm.

### (Example 8)

The procedure was performed in the same manner as in Example 5 except that a 50 g weight was dropped from a position at a height of 70 mm.

### (Comparative Example 1)

A PEG method, a method known in the art, was performed as Comparative Example 1. The pellet produced according to "Method for Mixing Cells" described above was subjected to the following operations. The tip of a pipet was placed at the time of pelleting, and the pellet and PEG1500 were mixed by slowly performing the following at the same time: an operation of ejecting 100 µL of PEG1500 (polyethylene glycol with a number average molecular weight of 1500, Roche) like gently placing it; and an operation like drawing a circle with the tip of the pipet. Then, the mixture was immersed in a water bath at 37°C and slowly shaken for 90 seconds. To the mixture, 100 µL of RPMI1640 pre-warmed to 37°C was slowly added, and the liquid was mixed by lightly shaking the tube. RPMI1640 was further added in stepwise increments of the amount to 300 µL and 1000 µL (both added slowly), and the liquid was mixed by lightly shaking the tube. After the mixture was reacted in a CO₂ incubator (37°C, 95% CO₂) for 10 minutes, the mixture was centrifuged (500 x g for 10 minutes) to remove the supernatant. The cells were suspended in PBS, and a fused cell liquid was formed.

Then, the cell fusion rate and the cell survival rate were each measured.

The results are shown in Tables 1 and 2. The results confirmed that cell fusion occurred by the cell fusion method and the cell fusion apparatus. The results also confirmed that the cell fusion rate was higher than the PEG method, the method known in the art.

**Table 1**

| | ZPP amount (mg) | Time until reaching maximum pressure (milliseconds) | Maximum pressure (MPa) | Cell fusion rate (%) | Cell survival rate (%) |
|---|---|---|---|---|---|
| Example 1 | 15 | 0.31 | 2.51 | 16.95 | 41.25 |
| Example 2 | 25 | 0.40 | 6.26 | 18.59 | 54.88 |
| Example 3 | 45 | 0.48 | 11.50 | 19.04 | 48.25 |
| Example 4 | 110 | 0.54 | 30.74 | 19.13 | 32.75 |
| Comparative Example 1 | - | - | - | 8.10 | 46.05 |

**Table 2**

| | Weight (g) | Position for dropping (mm) | Time until reaching maximum pressure (milliseconds) | Maximum pressure (MPa) | Cell fusion rate (%) |
|---|---|---|---|---|---|
| Example 5 | 50 | 30 | 0.64 | 1.75 | 9.34 |
| Example 6 | 300 | 20 | 2.0 | 6.12 | 8.92 |
| Example 7 | 300 | 10 | 1.70 | 5.35 | 10.50 |
| Example 8 | 50 | 70 | 0.80 | 5.35 | 11.52 |

### EXPLANATION OF REFERENCES

1 Injector; 2 Housing; 3 Syringe portion; 4 Plunger; 5 Piston; 6 Injector main body; 7 Drive portion; 8 Button; 9 Battery; 10 Injector assembly; 31 Nozzle portion: 31a Injection port; 32 Container portion; 41 Cap; 42 Fixing portion; 43 Sealing portion; 71 Igniter; 10A Injector assembly; 11 Holder; 51 Weight receiving portion; 61 Outer circumferential surface of injector body; 62 Flange; 100 Falling weight tester; 110 Bed; 110A Upper surface of bed; 111 Leg portion; 120 Support plate; 120A Upper surface of support plate; 120B Lower surface of support plate; 121 Holding hole; 121A Holding recessed portion; 121B Small-diameter hole portion; 130 Support column; 140 Frame; 141 Back frame; 142 Side frame; 143 Insertion hole; 150 Guide rod; 160 Weight; 160A Upper surface of weight; 160B Lower surface of weight

## Claims

1. A cell fusion method comprising:
pressurizing, by a pressurization portion, a solution including multiple cells of one or more types and at least one type of chemical cell fusion agent, the solution contained in a container portion.

2. The method according to claim 1, wherein
in pressurization by the pressurization portion in the pressurizing, time from start of the pressurization until a pressure in the container portion reaches a maximum pressure is 2.0 milliseconds or less, and the maximum pressure is 1.75 MPa or greater and 30.74 MPa or less.

3. The method according to claim 1 or 2, wherein the at least one type of chemical cell fusion agent is polyethylene glycol.

4. A cell fusion apparatus comprising:
a container portion configured to contain a solution including multiple cells of one or more types and at least one type of chemical cell fusion agent; and
a pressurization portion configured to pressurize the solution contained in the container portion upon actuation.

5. The apparatus according to claim 4, wherein
in pressurization by the pressurization portion, time from start of the pressurization until a pressure in the container portion reaches a maximum pressure is 2.0 milliseconds or less, and the maximum pressure is 1.75 MPa or greater and 30.74 MPa or less.
